# EUROPEAN PATENT APPLICATION

(11) **EP 4 310 094 A1**
(43) Date of publication of application: **24.01.2024**
(21) Application number: 22771542.2
(22) Date of filing: 18.03.2022
(51) Int. Cl.: C07K 1/06, C07D 233/60, C07K 1/02

(54) **METHOD FOR PRODUCING AMINO ACID OR PEPTIDE, REAGENT FOR FORMING PROTECTING GROUP, AND COMPOUND**

(30) Priority: 19.03.2021 JP 2021045625
(71) Applicant: FUJIFILM Corporation, Tokyo 106-8620 (JP)
(72) Inventor: NAKAGAWA Daisuke, Ashigarakami-gun, Kanagawa 258-8577 (JP); TSUYAMA Hiroaki, Ashigarakami-gun, Kanagawa 258-8577 (JP)
(74) Representative: Hoffmann Eitle
(86) International application number: PCT/JP2022/012551
(87) International publication number: WO 2022/196797

(57) **Abstract**

An object of the present invention is to provide a method for producing a peptide with high efficiency, which is capable of deprotecting a protective group in a side chain with a weak acid; a protective group-forming reagent having excellent deprotective properties; and a compound. According to the present invention, there is provided a method for producing a peptide, including a peptide chain extending step of reacting a first amino acid or peptide in which a side chain is protected with a first protective group represented by Formula (1) with a second amino acid or peptide to obtain a third amino acid or peptide, and a first deprotecting step of deprotecting the first protective group of the third amino acid or peptide, in which, in the first deprotecting step, the deprotection is performed using a deprotection solution having a trifluoroacetic acid content of 10% by mass or less. In the formula, R₁₁ is an aryl group having a substituent, R₂₁ and R₂₂ are each independently a hydrogen atom or a substituent, and n is an integer of 1 to 6.

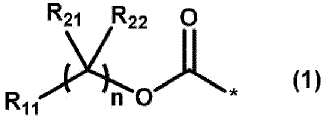

## Description

### Technical Field

The present disclosure relates to a method for producing an amino acid or a peptide, a protective group-forming reagent, and a compound.

### Background Art

Examples of a method for producing a peptide include a solid phase method and a liquid phase method.

The liquid phase method is advantageous in that good reactivity is exhibited, and intermediate peptide can be purified by extraction and washing, isolation, and the like after a condensation reaction. However, depending on the peptide to be synthesized, the performance of an existing side chain protective group may be insufficient.

As a protective group-forming reagent in the related art, compounds disclosed in Patent Document 1 and Patent Document 2 have been known.

### Prior Art Documents

Patent Documents

Patent Document 1: JP1990-36197A(JP-H2-36197A)
Patent Document 2: JP1992-360899A (JP-4-360899A)

### SUMMARY OF THE INVENTION

However, the protective groups disclosed in Patent Documents 1 and 2 have insufficient performance.

An object to be achieved by an embodiment of the present disclosure is to provide a method for producing an amino acid or a peptide with high efficiency, which is capable of deprotecting a protective group in a side chain with a weak acid.

An object to be achieved by another embodiment of the present disclosure is to provide a protective group-forming reagent having excellent deprotective properties and a compound protected by the protective group-forming reagent.

The methods for achieving the above-described objects include the following aspects.
<1> A method for producing a peptide, comprising:
   a peptide chain extending step of reacting a first amino acid or peptide in which a side chain is protected with a first protective group represented by Formula (1) with a second amino acid or peptide to obtain a third amino acid or peptide; and
   a first deprotecting step of deprotecting the first protective group of the third amino acid or peptide,
   in which, in the first deprotecting step, the deprotection is performed using a deprotection solution having a trifluoroacetic acid content of 10% by mass or less, 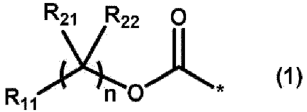 in the formula, R₁₁ is an aryl group having a substituent, R₂₁ and R₂₂ are each independently a hydrogen atom or a substituent, n is an integer of 1 to 6, and an asterisk represents a linking portion.
<2> The method for producing a peptide according to <1>, in which the trifluoroacetic acid content of the deprotection solution is 2% by mass or less.
<3> The method for producing a peptide according to <1> or <2>, further comprising:
   a C-terminal protecting step of protecting a carboxy group or an amide group of an N-terminal protected amino acid or peptide with a C-terminal protective agent;
   an N-terminal deprotecting step of deprotecting an N-terminal protective group of an N-terminal and C-terminal protected amino acid or peptide obtained in the C-terminal protecting step;
   a peptide chain extending step of condensing an N-terminal of a C-terminal protected amino acid or peptide obtained in the N-terminal deprotecting step with an N-terminal protected amino acid or peptide; and
   a C-terminal deprotecting step of deprotecting a C-terminal protective group,
   in which any of the C-terminal protected amino acid or peptide or the N-terminal protected amino acid or peptide is the first amino acid or peptide protected by the first protective group.
<4> The method for producing a peptide according to any one of <1> to <3>,
   in which the substituent of the aryl group in R₁₁ is an electron-donating group, and
   a plurality of substituents may be the same or different from each other.
<5> The method for producing a peptide according to <4>,
   in which the electron-donating group is an amino group, an alkoxyalkyl group, an alkoxy group, or an alkyl group, which may further have a substituent.
<6> The method for producing a peptide according to <4>,
   in which the electron-donating group is an alkoxy group having 1 to 10 carbon atoms.
<7> The method for producing a peptide according to any one of <1> to <6>,
   in which the aryl group is a phenyl group or a naphthyl group.
<8> The method for producing a peptide according to any one of <1> to <7>,
   in which the first protective group is a protective group for a guanidino group of arginine.
<9> A method for producing an amino acid or a peptide, comprising:
   a protecting step of reacting a first amino acid or peptide with a protective group-forming reagent represented by Formula (2) to obtain a first protected amino acid or peptide in which a side chain is protected with a first protective group, 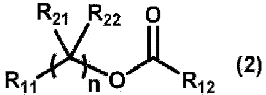 in the formula, R₁₁ is an aryl group having a substituent, R₂₁ and R₂₂ are each independently a hydrogen atom or a substituent, and n is an integer of 1 to 6.
<10> Arginine or a compound containing an arginine residue, which has a protective group represented by Formula (1), 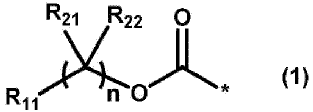 in the formula, R₁₁ is an aryl group having an electron-donating group, where a plurality of electron-donating groups may be the same or different from each other, R₂₁ and R₂₂ are each independently a hydrogen atom or a substituent, n is an integer of 1 to 6, and an asterisk represents a linking portion.
<11> The arginine or the compound containing an arginine residue according to <10>,
   in which the electron-donating group is an amino group, an alkoxyalkyl group, an alkoxy group, or an alkyl group, which may have a substituent.
<12> The arginine or the compound containing an arginine residue according to <11>,
   in which the electron-donating group is an alkoxy group having 1 to 10 carbon atoms.
<13> The arginine or the compound containing an arginine residue according to any one of <10> to <12>,
   in which the aryl group is a phenyl group or a naphthyl group.
<14> The arginine or the compound containing an arginine residue according to any one of <10> to <13>,
   in which the protective group is a protective group for a guanidino group of arginine.
<15> The arginine or the compound containing an arginine residue according to any one of <10> to <14>,
   in which the arginine or the compound further has a 9-fluorenylmethoxycarbonyl group.
<16> A protective group-forming reagent represented by Formula (2), 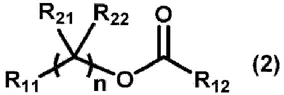 in the formula, R₁₁ is an aryl group which may have a substituent, R₁₂ is an aryl group which may have a substituent, a heteroaliphatic ring group, or a heteroaryl group, where the groups may be linked through an oxygen atom or a sulfur atom, R₂₁ and R₂₂ are each independently a hydrogen atom or a substituent, and n is an integer of 1 to 6.

According to an embodiment of the present invention, it is possible to provide a method for producing a peptide with high efficiency, which is capable of deprotecting a protective group in a side chain with a weak acid.

In addition, according to another embodiment of the present invention, it is possible to provide a protective group-forming reagent having excellent deprotective properties and a compound protected by the protective group-forming reagent.

### DESCRIPTION OF THE PREFERRED EMBODIMENTS

Hereinafter, the content of the present disclosure will be described in detail. The description of configuration requirements below is made based on representative embodiments of the present disclosure in some cases, but the present disclosure is not limited to such embodiments.

In the present specification, unless otherwise specified, each term has the following meaning.

A numerical range represented using "to" means a range including numerical values described before and after the preposition "to" as a lower limit value and an upper limit value.

In numerical ranges described in stages in the present specification, an upper limit value or a lower limit value described in one numerical range may be replaced with an upper limit value or a lower limit value of a numerical range described in another stage. In addition, in the numerical ranges described in the present specification, the upper limit value or the lower limit value of the numerical ranges may be replaced with the values shown in examples.

The term "step" includes not only the independent step but also a step in which intended purposes are achieved even in a case where the step cannot be precisely distinguished from other steps.

In a case where substitution or unsubstitution is not noted in regard to the notation of a "group" (atomic group), the "group" includes not only a group not having a substituent but also a group having a substituent. For example, the concept of an "alkyl group" includes not only an alkyl group not having a substituent (unsubstituted alkyl group) but also an alkyl group having a substituent (substituted alkyl group).

A chemical structural formula may be described by a simplified structural formula in which hydrogen atoms are omitted.

"% by mass" has the same definition as that for "% by weight", and "part by mass" has the same definition as that for "part by weight".

A combination of two or more preferred aspects is a more preferred aspect.

"Alkyl group" may be chain-like or branched, and may be substituted with a halogen atom or the like.

The "alkyl group" is preferably an alkyl group having 1 to 10 carbon atoms (also referred to as "the number of carbon atoms"). The number of carbon atoms in the alkyl group is more preferably 1 to 6, still more preferably 1 to 4, and even more preferably 1 or 2. Specific examples thereof include methyl, ethyl, propyl, isopropyl, butyl, and tert-butyl.

"Alkenyl group" is preferably an alkenyl group having 2 to 6 carbon atoms. Specific examples thereof include 1-propenyl.

As "aryl group", an aryl group having 6 to 14 carbon atoms is preferable, and examples thereof include a phenyl group, a 1-naphthyl group, a 2-naphthyl group, a biphenylyl group, and a 2-anthryl group. Among these, an aryl group having 6 to 10 carbon atoms is more preferable, and a phenyl group is particularly preferable.

Examples of "silyl group" include trimethylsilyl, triethylsilyl, dimethylphenylsilyl, tert-butyldimethylsilyl, and tert-butyldiethylsilyl.

Examples of "halogen atom" include a fluorine atom, a chlorine atom, a bromine atom, and an iodine atom.

As "alkoxy group", an alkoxy group having 1 to 10 carbon atoms is preferable. The number of carbon atoms in the alkyl group is more preferably 1 to 6, still more preferably 1 to 4, and even more preferably 1 or 2. Specific examples thereof include methoxy, ethoxy, and propoxy.

As "arylalkyl group", an arylalkyl group having 7 to 20 carbon atoms is preferable. The number of carbon atoms is preferably 7 to 12. Specific examples thereof include benzyl.

As "heteroarylalkyl group", a heteroarylalkyl group having 6 to 20 carbon atoms is preferable. The number of carbon atoms is preferably 6 to 12.

As "acyl group", an acyl group having 1 to 6 carbon atoms is preferable. Specific examples thereof include acetyl and propionyl.

As "arylalkylcarbonyl group", an arylalkylcarbonyl group having 7 to 10 carbon atoms is preferable. Specific examples thereof include benzylcarbonyl.

As "alkoxycarbonyl group", an alkoxycarbonyl group having 1 to 6 carbon atoms is preferable. Specific examples thereof include methoxycarbonyl, ethoxycarbonyl, and a Boc group. The Boc group means a tert-butoxycarbonyl group.

As "arylalkyloxycarbonyl group", an arylalkyloxycarbonyl group having 7 to 20 carbon atoms is preferable. The number of carbon atoms is preferably 8 to 20 and more preferably 8 to 14. Specific examples thereof include benzyloxycarbonyl (hereinafter, also referred to as a Cbz group or a Z group) and an Fmoc group. The Fmoc group means a 9-fluorenylmethoxycarbonyl group.

In a case where the amino acid and peptide according to the present disclosure have a hydroxy group, an amino group (-NH₂ or -NHR; R represents an alkyl group or an aryl group), a carboxy group, a carbonyl group, an amide group, a guanidyl group, a mercapto group, or the like, these groups may be protected, and a target compound can be obtained by removing a protective group after the reaction, as necessary.

Examples of a protective group of the hydroxy group include an alkyl group, an aryl group, a trityl group, an arylalkyl group having 7 to 10 carbon atoms, a formyl group, an acyl group having 1 to 6 carbon atoms, a benzoyl group, an arylalkylcarbonyl group having 7 to 10 carbon atoms, a 2-tetrahydropyranyl group, a 2-tetrahydrofuranyl group, a silyl group, and an alkenyl group having 2 to 6 carbon atoms. These groups may be substituted with one to three substituents selected from the group consisting of a halogen atom, an alkyl group having 1 to 6 carbon atoms, an alkoxy group having 1 to 6 carbon atoms, and a nitro group.

Examples of a protective group of the amino group include a formyl group, an acyl group having 1 to 6 carbon atoms, an alkoxycarbonyl group having 1 to 6 carbon atoms, a benzoyl group, an arylalkylcarbonyl group having 7 to 10 carbon atoms, an arylalkyloxycarbonyl group having 7 to 14 carbon atoms, a trityl group, a monomethoxytrityl group, a 1-(4,4-dimethyl-2,6-dioxocyclohex-1-ylidene)-3-methylbutyl group, a phtaloyl group, an N,N-dimethylaminomethylene group, a silyl group, and an alkenyl group having 2 to 6 carbon atoms. These groups may be substituted with one to three substituents selected from the group consisting of a halogen atom, an alkoxy group having 1 to 6 carbon atoms, and a nitro group.

Examples of a protective group of the carboxy group include the above-described protective group of the hydroxy group and a trityl group.

Examples of a protective group of the amide group include a trityl group.

Examples of a protective group of the carbonyl group include cyclic acetals (for example, 1,3-dioxane) and acyclic acetals (for example, di(alkyl having 1 to 6 carbon atoms) acetal).

Examples of a protective group of the guanidino group include a 2,2,4,6,7-pentamethyldihydrobenzofuran-5-sulfonyl group, a 2,3,4,5,6-pentamethylbenzenesulfonyl group, a tosyl group, and a nitro group.

Examples of a protective group of the mercapto group (thiol group) include a trityl group, a 4-methylbenzyl group, an acetylaminomethyl group, a t-butyl group, and a t-butylthio group.

The method of removing the above-described protective group may be performed according to a known method described in, for example, Protective Groups in Organic Synthesis, John Wiley and Sons (1980). A method using acid, base, ultraviolet light, hydrazine, phenylhydrazine, sodium N-methyldithiocarbamate, tetrabutylammonium fluoride, palladium acetate, or trialkylsilyl halide, or a reduction method is used.

"Amino acid" is an α, β, or γ amino acid, and is not limited to a naturally occurring amino acid and may be a non-naturally occurring amino acid such as N-methyl amino acid. In addition, the "Amino acid" may be an amino acid analog such as hydroxycarboxylic acid.

(Protective group-forming reagent, and arginine or compound containing arginine residue)

The protective group-forming reagent according to the embodiment of the present disclosure is represented by Formula (2), and is preferably represented by Formula (2a). Such a compound is used in the method for producing an amino acid or a peptide according to the embodiment of the present invention.

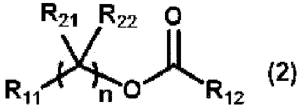

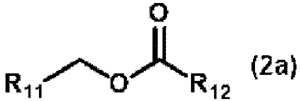

In Formulae (2) and (2a), R₁₁ is an aryl group which may have a substituent, R₂₁ and R₂₂ are each independently a hydrogen atom or a substituent, and n is an integer of 1 to 6.

The substituent of the aryl group in R₁₁ is preferably an electron-donating group, and the electron-donating group is preferably an amino group, an alkoxyalkyl group, an alkoxy group, or an alkyl group, in which these groups may have a substituent and a plurality of these groups may be the same or different from each other.

The electron-donating group is preferably an alkoxy group, more preferably an alkoxy group having 1 to 10 carbon atoms, and still more preferably an alkoxy group having 1 to 6 carbon atoms. The electron-donating group preferably has no substituent.

The aryl group is preferably a phenyl group or a naphthyl group.

As the substituent protected by the protective group-forming reagent, an amino group, an imino group, or a guanidino group (amino group and imino group) is preferable, and a guanidino group of arginine is more preferable.

R₁₂ is an aryl group which may have a substituent, a heteroaliphatic ring group which may have a substituent, or a heteroaryl group which may have a substituent, the groups may be linked through an oxygen atom (O) or a sulfur atom (S).

The aryl group is preferably a phenyl group or a naphthyl group.

The heteroaliphatic ring group is preferably a nitrogen-containing heteroaliphatic ring group, and more preferably a succinimide group.

The heteroaryl group is preferably a monocyclic nitrogen-containing heteroaryl group, and more preferably imidazolyl, pyridyl, pyrazolyl, or triazolyl. These may be linked by a nitrogen atom. The substituent of the aryl group, heteroaliphatic ring group, or heteroaryl group in R₁₂ is preferably an electron-withdrawing group, and a plurality of electron-withdrawing groups may be the same or different from each other. The electron-withdrawing group is preferably a carbonyl group, a nitro group, or a halogen atom, and more preferably a nitro group or a fluoro group.

Examples of the substituent represented by R₂₁ and R₂₂ include a halogen atom and an alkyl group.

Specific examples of the protective group-forming reagent are shown below. In the formulae, R represents an electron-donating group and m represents an integer of 0 to 5.

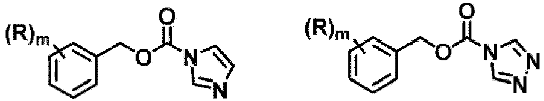

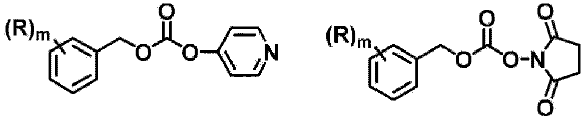

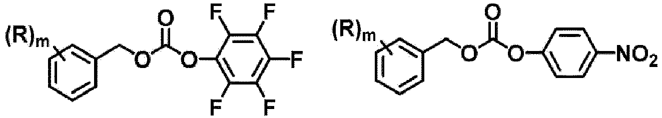

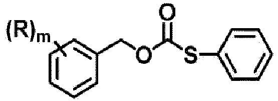

A compound protected by the above-described protective group-forming reagent is preferably arginine or a compound (preferably, a peptide) containing an arginine residue.

Specifically, the protected compound is arginine or a compound containing an arginine residue, which has a protective group represented by Formula (1), and the protected compound may further have an Fmoc protective group (9-fluorenylmethoxycarbonyl group). The protective group represented by Formula (1) is preferably a protective group represented by Formula (1a).

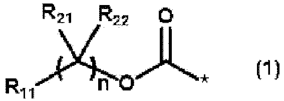

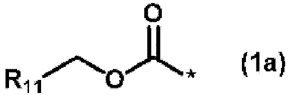

In the formula, R₁₁ is an aryl group which may have a substituent, R₂₁ and R₂₂ are each independently a hydrogen atom or a substituent, n is an integer of 1 to 6, and an asterisk in the formula represents a linking portion. In the formula, suitable aspects of R₁₁, R₂₁, and R₂₂ are the same as those in Formula (2), and the same applies to the following description.

### (Method for producing peptide)

In the method for producing a peptide according to the embodiment of the present disclosure, the protective group-forming reagent represented by Formula (2) is used. A step of using the above-described protective group-forming reagent represented by Formula (2) is preferably a side chain protecting step of protecting a side chain of an amino acid or a peptide. The amino acid is preferably arginine, and the peptide is preferably a peptide containing an arginine residue.

The protective group-forming reagent represented by Formula (2) can be used as a protective group for the amino acid, and is more preferably used as a protective group for a guanidino group, which is a side chain of the amino acid, an amino group, or an imino group, and still more preferably used as a protective group for a guanidino group.

From the viewpoint of ease of synthesis of the peptide and yield, it is more preferable that the method for producing a peptide according to the embodiment of the present disclosure further includes, in addition to the above-described side chain protecting step, an N-terminal deprotecting step of deprotecting an N-terminal of the N-terminal and C-terminal protected amino acid or N-terminal and C-terminal protected peptide obtained through the above-described side chain protecting step, and a peptide chain extending step of condensing an N-terminal protected amino acid or an N-terminal protected peptide to the N-terminal of the C-terminal protected amino acid or C-terminal protected peptide obtained in the above-described N-terminal deprotecting step;
it is still more preferable to further include a precipitating step of precipitating the N-terminal and C-terminal protected peptide obtained in the above-described peptide chain extending step; and
it is even more preferable to further include, one or more times in the following order, a step of, after the above-described precipitating step, deprotecting an N-terminal of the obtained N-terminal and C-terminal protected peptide, a step of condensing an N-terminal protected amino acid or an N-terminal protected peptide to the N-terminal of the obtained C-terminal protected peptide, and a step of precipitating the obtained N-terminal and C-terminal protected peptide.

According to a preferred aspect, the method for producing a peptide according to the embodiment of the present disclosure includes:
a C-terminal protecting step of protecting a carboxy group or an amide group of an N-terminal protected amino acid or peptide with a C-terminal protective agent;
an N-terminal deprotecting step of deprotecting an N-terminal protective group of an N-terminal and C-terminal protected amino acid or peptide obtained in the C-terminal protecting step;
a peptide chain extending step of condensing an N-terminal of a C-terminal protected amino acid or peptide obtained in the N-terminal deprotecting step with an N-terminal protected amino acid or peptide; and
a C-terminal deprotecting step of deprotecting a C-terminal protective group,
in which any of the C-terminal protected amino acid or peptide or the N-terminal protected amino acid or peptide is the first amino acid or peptide protected by the first protective group.

The N-terminal protected amino acid or the N-terminal protected peptide is an amino acid or a peptide in which only the N-terminal is protected, and the N-terminal and C-terminal protected amino acid or peptide is an amino acid or a peptide in which the N-terminal and the C-terminal are protected.

The formation reaction (condensation reaction) of the peptide bond between the amino group and the carboxy group and the deprotection of the protective group in each step can be carried out by a known method. For example, WO2020/175473A and WO2020/175472A are referred to, which are incorporated herein by reference.

Hereinafter, each step described above will be described in detail.

### <Side chain protecting step>

The method for producing an amino acid or a peptide according to the embodiment of the present disclosure preferably includes a side chain protecting step of protecting a side chain amino group of an amino acid or a peptide with the above-described protective group-forming reagent represented by Formula (2). That is, according to the present invention, a method for producing an amino acid or a peptide, including a protecting step of reacting the first amino acid or peptide with the above-described protective group-forming reagent represented by Formula (2) to obtain a first protected amino acid or peptide in which a side chain is protected with a first protective group, is provided.

The amino acid to be protected is preferably arginine, and a side chain, an amino group, or a carboxy group of another amino acid may be protected. In addition, in a case where the side chain of the peptide is protected, it is preferable that a side chain of a portion derived from arginine is protected.

In the side chain protecting step, the first amino acid or peptide in which the side chain is protected by the first protective group represented by Formula (1) is obtained. The present invention relates to a method for producing an amino acid or a peptide, which includes such a protecting step.

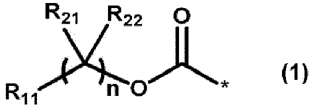

In the side chain protecting step, it is preferable to carry out the reaction under basic conditions in a solvent which does not affect the reaction.

As the solvent, a general organic solvent can be used in the reaction. Specific examples thereof include halogenated hydrocarbons such as chloroform and dichloromethane; and nonpolar organic solvents such as 1,4-dioxane, tetrahydrofuran (THF), and cyclopentyl methyl ether. Two or more kinds of these solvents may be mixed and used. In addition, in the above-described halogenated hydrocarbons or nonpolar organic solvents, aromatic hydrocarbons such as benzene, toluene, and xylene; nitriles such as acetonitrile and propionitrile; ketones such as acetone and 2-butanone; amides such as N,N-dimethylformamide and N-methylpyrrolidone (NMP); and sulfoxides such as dimethyl sulfoxide may be mixed and used. In particular, NMP is preferable.

Examples of the base include tertiary amines such as triethylamine and diisopropylethylamine, and non-nucleophilic organic bases such as 1,8-diazabicyclo[5.4.0]-7-undecene (DBU), 1,4-diazabicyclo[2.2.2]octane (DABCO), and 1,5-diazabicyclo[4.3.0]-5-nonene (DBN).

An amount of the base used is preferably more than 1 molar equivalent and 20 molar equivalent or less, and more preferably 2 molar equivalent to 10 molar equivalent with respect to 1 molar equivalent of the above-described protective group-forming reagent represented by Formula (2).

In a case of protecting the guanidino group of arginine, it is preferable to protect the amino group and carboxy group of the arginine with an allyloxycarbonyl group (Alloc), an allyl group (Allyl), or the like.

### <Side chain deprotecting step>

The method for producing a peptide according to the embodiment of the present invention includes a peptide chain extending step of reacting a first amino acid or peptide protected by the first protective group with a second amino acid or peptide to obtain a third amino acid or peptide; and
a first deprotecting step of deprotecting the first protective group of the third amino acid or peptide,
in which, in the first deprotecting step, the deprotection is performed using a deprotection solution having a trifluoroacetic acid (TFA) content of 10% by mass or less.

The TFA content of the deprotection solution is preferably 7% by mass or less, more preferably 5% by mass or less, still more preferably 2% by mass or less, still more preferably 1% by mass or less, still more preferably 0.5% by mass or less, and still more preferably 0.1% by mass or less. The TFA content of the deprotection solution is preferably 0.01% by mass or more, and more preferably 0.1% by mass or more. In a case where the TFA content is high, the obtained peptide is decomposed, by-products are produced, and production efficiency is lowered. In a case of being low, the deprotection takes time, and the production efficiency is lowered.

In the present invention, both deprotective properties with a weak acid and sealing property of the side chain (particularly, the guanidino group) can be achieved.

### <C-terminal protecting step>

The method for producing a peptide according to the embodiment of the present disclosure preferably includes a C-terminal protecting step of protecting a carboxy group or an amide group of the amino acid or the peptide (may be the first amino acid or peptide or the second amino acid or peptide described above) with a C-terminal protective group.

As the C-terminal protective group, a compound having an aliphatic hydrocarbon group having 12 or more carbon atoms is preferable, and the number of carbon atoms is preferably 15 or more and more preferably 20 to 30. In a case where the C-terminal protective group has a plurality of aliphatic hydrocarbon groups, the total number of carbon atoms therein is preferably 30 to 80 and more preferably 36 to 80. The C-terminal protective group preferably has a ring structure, and more preferably has a condensed polycycle, an aromatic heterocyclic ring, or a naphthalene ring.

As the C-terminal protective group, an aromatic heterocyclic compound represented by Formula (1) in WO2020/175473A is preferable. For such a compound, WO2020/175473A is referred to, which is incorporated herein by reference.

As the C-terminal protective group, a condensed polycyclic aromatic hydrocarbon compound represented by Formula (1) in WO2020/175472A is also preferable. For such a compound, WO2020/175472A is referred to, which is incorporated herein by reference.

The C-terminal protective group may be a compound represented by Formula (1) in WO2020/262259A, and WO2020/262259A is referred to, which is incorporated herein by reference.

The amino acid or peptide used in the above-described C-terminal protecting step is not particularly limited, but is preferably an N-terminal protected amino acid or an N-terminal protected peptide, and more preferably an Fmoc-protected amino acid or an Fmoc-protected peptide.

In addition, it is preferable that a hydroxy group, an amino group, a carbonyl group, a carboxyl group, an amide group, an imidazole group, an indole group, a guanidyl group, a mercapto group, or the like, which is a moiety other than the C-terminal end in the amino acid or peptide used in the above-described C-terminal protecting step, is protected by a protective group.

In a case where the bonding site of the C-terminal protective group is -OH or -SH, it is preferable to add a condensing agent in the presence of a condensation additive (condensation activating agent) in a solvent which does not affect the reaction, or to react in an acid catalyst.

In a case where the bonding site of the C-terminal protective group is -NHR¹⁸ (R¹⁸ is a hydrogen atom, an alkyl group, an arylalkyl group, or a heteroarylalkyl group), it is preferable to add a condensing agent in the presence of a condensation additive (condensation activating agent), or to react the condensing agent with a base.

As the solvent, condensation additive (condensation activating agent), condensing agent, acid catalyst, and base, WO2020/175472A, WO2020/175473A, and WO2020/262259A are referred to, which are incorporated herein by reference.

As the solvent, a nonpolar organic solvent is preferable, and THF is more preferable.

In addition, a solvent described in Organic Process Research & Development, 2017, 21, 3, 365 to 369 may be used.

As the condensing agent, a condensing agent generally used in peptide synthesis can be used without limitation in the present disclosure. Examples thereof include 4-(4,6-dimethoxy-1,3,5-triazin-2-yl)-4-methylmorpholinium chloride (DMT-MM), O-(benzotriazol-1-yl)-1,1,3,3-tetramethyluronium hexafluorophosphate (HBTU), O-(7-azabenzotriazol-1-yl)-1,1,3,3-tetramethyluronium hexafluorophosphate (HATU), O-(6-chlorobenzotriazol-1-yl)-1,1,3,3-tetramethyluronium hexafluorophosphate (HBTU(6-Cl)), O-(benzotriazol-1-yl)-1,1,3,3-tetramethyluronium tetrafluoroborate (TBTU), O-(6-chlorobenzotriazol-1-yl)-1,1,3,3 -tetramethyluronium tetrafluoroborate (TCTU), (1-cyano-2-ethoxy-2-oxoethylidenaminooxy)dimethylamino-morpholino-carbenium hexafluorophosphate (COMU), dicyclohexylcarbodiimide (DCC), diisopropylcarbodiimide (DIC), 1-ethyl-3-(3-dimethylaminopropyl) carbodiimide (EDC), a hydrochloride salt (EDC/HCl) of 1-ethyl-3-(3-dimethylaminopropyl) carbodiimide, and (benzotriazol-1-yloxy)tripyrrolidinophosphonium hexafluorophosphate (PyBoP).

Among these, DIC, EDC, EDC/HCl, DMT-MM, HBTU, HATU, or COMU is preferable.

An amount of the condensing agent to be used is preferably 1 molar equivalent to 10 molar equivalent and more preferably 1 molar equivalent to 5 molar equivalent with respect to 1 molar equivalent of the substrate.

As the acid catalyst used in the condensation reaction, an acid catalyst generally used in peptide synthesis can be used without limitation, and examples thereof include methanesulfonic acid, trifluoromethanesulfonic acid, p-toluenesulfonic acid, trifluoroacetic acid, and acetic acid.

Among these, methanesulfonic acid, p-toluenesulfonic acid, trifluoroacetic acid, or acetic acid is preferable.

An amount of the acid catalyst to be used is preferably 1 molar equivalent to 10 molar equivalent and more preferably 0.01 molar equivalent to 5 molar equivalent with respect to 1 molar equivalent of the substrate.

In the above-described C-terminal protecting step, it is preferable to add the condensation activating agent in order to promote the reaction and suppress side reactions such as racemization.

The condensation activating agent is a reagent which, in a case of coexisting with the condensing agent, leads an amino acid to a corresponding active ester, symmetric acid anhydride, or the like to facilitate the formation of a peptide bond (amide bond).

As the condensation activating agent, an activating agent generally used in peptide synthesis can be used without limitation, and examples thereof include 4-dimethylaminopyridine, N-methylimidazole, boronic acid derivative, 1-hydroxybenzotriazole (HOBt), ethyl 1-hydroxytriazole-4-carboxylate (HOCt), 1-hydroxy-7-azabenzotriazole (HOAt), 3-hydroxy-1,2,3-benzotriazin-4(3H)-one (HOOBt), N-hydroxysuccinimide (HOSu), N-hydroxyphthalimide (HOPht), N-hydroxy-5-norbornene-2,3-dicarboxyimide (HONb), pentafluorophenol, and ethyl(hydroxyimino)cyanoacetylate (Oxyma). Among these, 4-dimethylaminopyridine, HOBt, HOCt, HOAt, HOOBt, HOSu, HONb, or Oxyma is preferable.

An amount of the condensation activating agent to be used is preferably more than 0 molar equivalent and 4.0 molar equivalent or less and more preferably 0.1 molar equivalent to 1.5 molar equivalent with respect to 1 molar equivalent of the substrate.

As the solvent, the above-described solvent used in the above-described dissolving step can be suitably used.

The reaction temperature is not particularly limited, but is preferably -10°C to 80°C and more preferably 0°C to 40°C. The reaction time is not particularly limited, but is preferably 1 hour to 30 hours.

In addition, the N-terminal and C-terminal protected amino acid or N-terminal and C-terminal protected peptide obtained by the above-described C-terminal protecting step may be purified.

For example, in order to isolate the product obtained after dissolving the obtained N-terminal and C-terminal protected amino acid or N-terminal and C-terminal protected peptide in a solvent (reaction solvent, for example, THF) and performing a desired organic synthesis reaction. Thereafter, the solvent is changed to a solvent in which the N-terminal and C-terminal protected amino acid or N-terminal and C-terminal protected peptide is dissolved (for example, change of solvent composition or change of solvent type) to reprecipitate the resultant.

Specifically, the reaction is performed under conditions such that the N-terminal and C-terminal protected amino acid or N-terminal and C-terminal protected peptide is dissolved. After the reaction, the solvent is distilled off and then replaced, or after the reaction, by adding a polar solvent to the reaction system without distilling off the solvent, aggregates are precipitated and impurities are eliminated.

As the solvent for replacement or the polar solvent, polar organic solvents such as methanol, acetonitrile, and water are used alone or in combination. That is, the reaction is performed under conditions such that the N-terminal and C-terminal protected amino acid or N-terminal and C-terminal protected peptide is dissolved, and in the solvent replacement after the reaction, for example, a halogenated solvent, THF, or the like is used for dissolution, and a polar organic solvent such as methanol, acetonitrile, and water is used for precipitation.

### <N-terminal deprotecting step>

The method for producing a peptide according to the embodiment of the present disclosure preferably includes an N-terminal deprotecting step of deprotecting an N-terminal protective group of the N-terminal and C-terminal protected amino acid or N-terminal and C-terminal protected peptide obtained in the above-described C-terminal protecting step.

As the N-terminal protective group, a protective group for an amino group described later, which is generally used in a technical field such as peptide chemistry, can be used. In the present disclosure, a Boc group, a Cbz group, or an Fmoc group is suitable.

The deprotection conditions are appropriately selected depending on the type of the temporary protective group. For example, in a case of the Fmoc group, the deprotection is performed by treating with a base, and in a case of the Boc group, the deprotection is performed by treating with an acid. The reaction is performed in a solvent which does not affect the reaction.

Examples of the base include secondary amines such as dimethylamine and diethylamine, and non-nucleophilic organic bases such as 1,8-diazabicyclo[5.4.0]-7-undecene (DBU), 1,4-diazabicyclo[2.2.2]octane (DABCO), and 1,5-diazabicyclo[4.3.0]-5-nonene (DBN).

As the solvent, the above-described solvent used in the above-described dissolving step can be suitably used.

### <Peptide chain extending step>

The method for producing a peptide according to the embodiment of the present disclosure preferably includes a peptide chain extending step of condensing an N-terminal of a C-terminal protected amino acid or peptide obtained in the N-terminal deprotecting step with an N-terminal protected amino acid or peptide. In any of the C-terminal protected amino acid or peptide or the N-terminal protected amino acid or peptide, the side chain is protected by the above-described first protective group. The first protective group is then deprotected at an appropriate timing.

The above-described peptide chain extending step is preferably performed under peptide synthesis conditions generally used in the field of peptide chemistry, in which the above-described condensing agent, condensation additive, and the like are used.

The N-terminal protected amino acid or the N-terminal protected peptide is not particularly limited, and an Fmoc-protected amino acid or an Fmoc-protected peptide is suitable.

### <Precipitating step>

It is preferable that the method for producing a peptide according to the embodiment of the present disclosure further includes a precipitating step of precipitating the N-terminal and C-terminal protected peptide obtained in the above-described peptide chain extending step.

The precipitating step can be performed in the same manner as in the purification (reprecipitation) of the C-terminal protecting step described above.

Specifically, a polar solvent is added to the reaction system without distilling off the reaction solvent after the reaction in the previous stage. In this case, in the reaction solvent, THF is used as the nonpolar organic solvent and acetonitrile is used as the polar solvent. The proportion (volume basis) of the nonpolar organic solvent and the polar solvent used is preferably 1: 1 to 1:100, more preferably 1:3 to 1:50, and still more preferably 1:5 to 1:20. In the case of this proportion used, the N-terminal and C-terminal protected amino acid or N-terminal and C-terminal protected peptide can be efficiently precipitated, and the target product can be efficiently purified.

### <Chain extension>

It is preferable that the method for producing a peptide according to the embodiment of the present disclosure further includes, one or more times in the following order after the above-described precipitating step, a step of deprotecting the N-terminal of the obtained N-terminal and C-terminal protected peptide, a step of condensing the N-terminal of the obtained C-terminal protected peptide with an N-terminal protected amino acid or an N-terminal protected peptide, and a step of precipitating the obtained N-terminal and C-terminal protected peptide.

By repeating the above-described three steps, the chain extension of the obtained peptide can be easily performed.

Each step in the above-described three steps can be performed in the same manner as each corresponding step described above.

### <C-terminal deprotecting step>

It is preferable that the method for producing a peptide according to the embodiment of the present disclosure further includes a C-terminal deprotecting step of deprotecting a C-terminal protective group.

In the above-described C-terminal deprotecting step, by removing the above-described C-terminal protective group in the C-terminal protected peptide having a desired number of amino acid residues, a peptide as a final target product can be obtained. Preferred examples of a method of removing the C-terminal protective group include a deprotecting method using an acidic compound.

Examples thereof include a method of adding an acid catalyst and a hydrogenating method using a metal catalyst. Examples of the acid catalyst include trifluoroacetic acid (TFA), hydrochloric acid, trifluoroethanol (TFE), hexafluoroisopropanol (HFIP), and acetic acid. TFA is preferable for peptides which do not decompose with strong acids, and TFE, HFIP, or acetic acid is preferable for peptides which decompose with strong acids. A concentration of the acid can be appropriately selected depending on the side chain protective group of the extending amino acid and the deprotection conditions.

For example, a concentration of TFA is preferably 50% by volume or less, more preferably 30% by volume or less, still more preferably 10% by volume or less, even more preferably 5% by volume or less, and particularly preferably 1% by volume or less with respect to the total volume of the solvent used. The lower limit value thereof is preferably 0.01% by volume, more preferably 0.1% by volume, and still more preferably 0.5% by volume.

The deprotection time is preferably 5 hours or less, more preferably 3 hours or less, and still more preferably 1 hour or less.

In the present disclosure, the C-terminal protective group and the side chain protective group can be deprotected even under weak acid conditions, and a side reaction of the obtained peptide can be suppressed.

Examples of peptide which is suitable for deprotection of the C-terminal protective group under weak acid conditions (that is, peptide which is sensitive to acid) include peptides having an N-alkylamide structure.

From the viewpoint of suppressing side reactions of the obtained peptide and of temporal stability, the method for producing a peptide according to the embodiment of the present disclosure is preferably applied to a method for producing a peptide which is sensitive to acid, more preferably applied to a method for producing a peptide having an N-alkylamide structure.

### <Peptide>

The peptide, which is the final target product obtained by the method for producing a peptide according to the embodiment of the present disclosure, can be isolated and purified according to a method commonly used in peptide chemistry. For example, the peptide, which is the final target product, can be isolated and purified by extraction and washing the reaction mixture, crystallization, chromatography, and the like.

The type of peptide produced by the method for producing a peptide according to the embodiment of the present disclosure is not particularly limited, but it is preferable that the number of amino acid residues of the peptide is, for example, approximately several tens or less (specifically, 50 or less, 40 or less, 30 or less, or 20 or less). Same as existing or unknown synthetic or native peptides, the peptide obtained by the method for producing a peptide according to the embodiment of the present disclosure can be used in various fields such as pharmaceuticals, foods, cosmetics, electronic materials, biosensors, and the like, but the use of the peptide is not limited thereto.

In the method for producing a peptide according to the embodiment of the present disclosure, the precipitating step can be appropriately omitted as long as it does not affect the reaction in the next step.

### Examples

Hereinafter, the embodiments of the present invention will be more specifically described with reference to Examples. The materials, amounts to be used, proportions, treatment contents, treatment procedures, and the like shown in Examples can be appropriately modified as long as the modifications do not depart from the spirit of the embodiments of the present invention. Therefore, the scope of the embodiments of the present invention is not limited to the following specific examples. In addition, "parts" and "%" are on a mass basis unless otherwise specified. The room temperature means 25°C. Me in the chemical formula represents a methyl group.

Unless otherwise specified, purification by column chromatography was performed using an automatic purification device ISOLERA (manufactured by Biotage Ltd.) or a medium pressure liquid chromatograph YFLC-Wprep 2XYN (manufactured by YAMAZEN).

Unless otherwise specified, SNAPKP-SIl Cartridge (manufactured by Biotage Ltd.) or a high flash column W001, W002, W003, W004, or W005 (manufactured by YAMAZEN) was used as a carrier in silica gel column chromatography.

The mixing ratio in an eluent used for column chromatography is the volume ratio. For example, "gradient elution of hexane:ethyl acetate = 50:50 to 0:100" means that an eluent of 50% hexane and 50% ethyl acetate is finally changed to an eluent of 0% hexane and 100% ethyl acetate.

In addition, "gradient elution of hexane:ethyl acetate = 50:50 to 0:100 and gradient elution of methanol:ethyl acetate = 0:100 to 20:80" means that an eluent of 50% hexane and 50% ethyl acetate is changed to an eluent of 0% hexane and 100% ethyl acetate, and then the eluent of 0% hexane and 100% ethyl acetate is finally changed to an eluent of 20% methanol and 80% ethyl acetate.

MS spectrum was measured using ACQUITY SQD LC/MS System (manufactured by Waters Corporation; ionization method; electrospray ionization (ESI) method).

NMR spectrum was measured using Bruker AV300 (manufactured by Bruker, 300 MHz) or Bruker AV400 (manufactured by Bruker, 400 MHz), using tetramethylsilane as an internal reference, and all δ values were represented in ppm.

The HPLC purity was measured using ACQUITY UPLC (manufactured by Waters Corporation, column: CSH C18 1.7 µm).

### <Example>

### <Synthesis of protective group-forming reagent (1)>

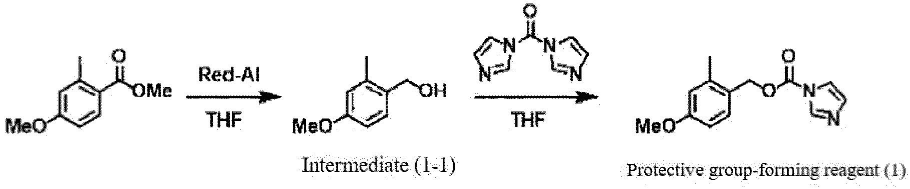

### <Synthesis of intermediate (1-1)>

In an ice bath, a toluene solution of 70% sodium bis(2-methoxyethoxy)aluminum hydride (20.2 g, 122.5 mmol) was added dropwise to a solution of methyl 4-methoxy-2-methylbenzoate (5.00 g, 27.2 mmol) in tetrahydrofuran (25 ml), and the mixture was stirred at room temperature for 30 minutes. After addition of acetone (3 ml) to the reaction solution, an aqueous solution (50 mL) of 20% potassium sodium tartrate and ethyl acetate (50 mL) were added thereto, and the mixture was stirred for 10 minutes. The organic layer was separated, and then concentrated under reduced pressure to obtain 4.81 g of a colorless oily intermediate (1-1).

### <Synthesis of protective group-forming reagent (1)>

In an ice bath, 1,1'-carbonyldiimidazole (4.70 g, 29.0 mmol) was added to a solution of the intermediate (1-1) (4.20 g, 27.6 mmol) in tetrahydrofuran (42 ml), and the mixture was stirred at room temperature for 30 minutes. After concentrating the reaction solution, a mixed solution (42 mL) of hexane/ethyl acetate = 1/1 and water (42 mL) were added thereto, and the mixture was stirred for 5 minutes. The organic layer was washed successively with water (42 mL) and saturated saline (10 mL), dried over sodium sulfate, and filtered. The filtrate was concentrated under reduced pressure to obtain 6.50 g of a protective group-forming reagent (1-1) (86% in two steps).

¹H-NMR (DMSOd₆, 400 MHz): δ = 8.13 to 8.10 (1H, m), 7.40 (1H, t, J = 1.5 Hz), 7.33 (1H, d, J = 8.4 Hz), 7.05 (1H, dd, 1.5, 0.9 Hz), 6.79 (1H, d, J = 2.5 Hz), 6.75 (1H, dd, 8.4, 2.8 Hz), 5.40 (2H, s), 3.81 (3H, s), 2.41 (3H, s)

### <Synthesis of protective group-forming reagents (2), (3), and (4)>

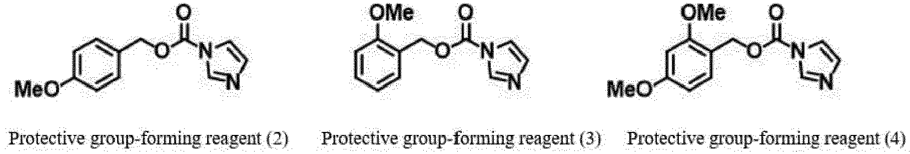

### <Protective group-forming reagent (2)>

The synthesis was carried out in the same manner as in the protective group-forming reagent (1), except that the intermediate (1-1) was changed to 4-methoxybenzyl alcohol.

### <Protective group-forming reagent (3)>

The synthesis was carried out in the same manner as in the protective group-forming reagent (1), except that the intermediate (1-1) was changed to 2-methoxybenzyl alcohol.

### <Protective group-forming reagent (4)>

The synthesis was carried out in the same manner as in the protective group-forming reagent (1), except that the intermediate (1-1) was changed to 2,4-dimethoxybenzyl alcohol.

### <Synthesis of intermediate (2-3)>

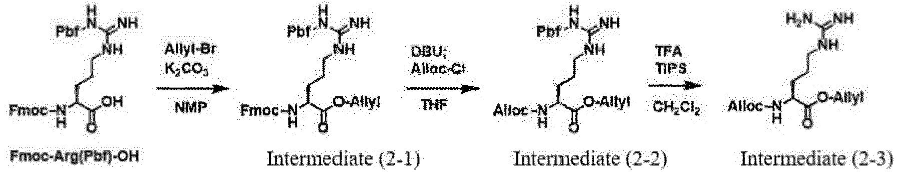

### <Synthesis of intermediate (2-1)>

At room temperature, allyl bromide (5.99 mL, 69.4 mmol) and potassium carbonate (19.2 g, 138 mmol) were added to a solution of Fmoc-Arg(Pbf)-OH (30.0 g, 46.2 mmol) in N-methylpyrrolidone (150 ml), and the mixture was stirred for 5 hours. Water (150 mL) and ethyl acetate (300 mL) were added to the reaction solution to separate the organic layer, and the organic layer was washed twice with water (100 mL) and with saturated saline (50 mL). After drying with sodium sulfate and filtering, the filtrate was concentrated under reduced pressure to obtain 31.2 g of a pale yellow solid intermediate (2-1).
ESI-MS(+) = 689.4

### <Synthesis of intermediate (2-2)>

At room temperature, diazabicycloundecene (6.77 g, 45.3 mmol) was added to a solution of the intermediate (2-1) (31.2 g, 45.3 mmol) in tetrahydrofuran (150 ml), and the mixture was stirred at room temperature for 30 minutes. N-(allyloxycarbonyloxy)succinimide (9.0 mL, 45.3 mmol) was added to the reaction solution, the mixture was stirred for 1.5 hours, and then 1 mol/L hydrochloric acid (100 mL) and ethyl acetate (300 mL) were added thereto. The organic layer was washed successively with water (100 mL), saturated aqueous sodium bicarbonate (100 mL), and saturated saline (10 mL), dried over sodium sulfate, and filtered. The filtrate was concentrated under reduced pressure, and the obtained residue was purified by silica gel column chromatography (eluent: hexane/ethyl acetate) to obtain 18.3 g of an intermediate (2-2) (73% in two steps).
ESI-MS(+) = 551.3

### <Synthesis of intermediate (2-3)>

At room temperature, triisopropylsilane (9.3 mL, 45.4 mmol) and trifluoroacetic acid (57 mL) were added to a solution of the intermediate (2-2) (12.5 g, 22.7 mmol) in methylene chloride (57 ml), and the mixture was stirred for 1 hour. The reaction solution was concentrated, methyl tert-butyl ether (100 mL) was added thereto, and the mixture was suspended and stirred for 30 minutes. The solid substance was collected by filtration to obtain 8.6 g of a pale yellow intermediate (2-3) as a trifluoroacetate.
ESI-MS(+) = 298.8

### <Synthesis method of example compound 1>

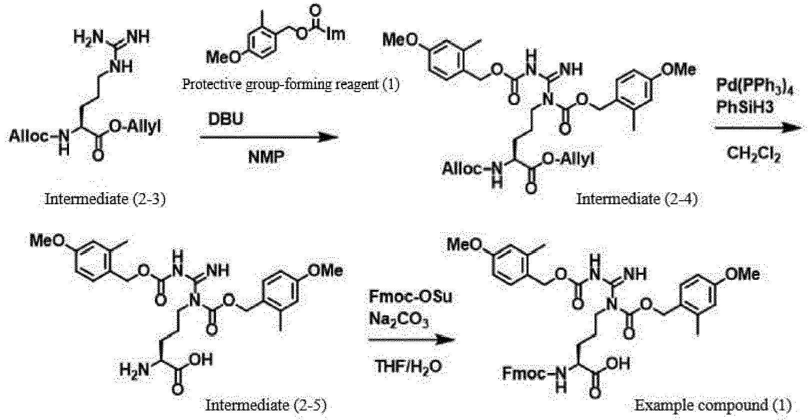

### <Synthesis of intermediate (2-4)>

At room temperature, diazabicycloundecene (14.7 mL, 98.4 mmol) and the protective group-forming reagent (1) (29.1 g, 118 mmol) were added to a solution of the intermediate (2-3) (7.80 g, 19.7 mmol) in N-methylpyrrolidone (39 ml), and the mixture was stirred at room temperature for 4 hours. Saturated ammonium chloride aqueous solution (40 mL) and ethyl acetate (80 mL) were added to the reaction solution, and the organic layer was washed twice with water (40 mL) and saturated saline (10 mL) successively, dried over sodium sulfate, and filtered. The filtrate was concentrated under reduced pressure, and the obtained residue was purified by silica gel column chromatography (eluent: hexane/ethyl acetate) to obtain 5.03 g of an intermediate (2-4).

### <Synthesis of intermediate (2-5)>

At room temperature, triphenylsilane (1.1 mL, 9.16 mmol) and tetrakis triphenylphosphine palladium (0.265 g, 5 mol%) were added to a solution of the intermediate (2-4) (3.00 g, 4.58 mmol) in methylene chloride (30 ml), and the mixture was stirred for 1 hour. The reaction solution was concentrated under reduced pressure to obtain 2.00 g of an intermediate (2-5).
ESI-MS(+) = 531.1

### <Synthesis of example compound (1)>

At room temperature, sodium carbonate (1.32 g, 12.4 mmol) and N-(9-fluorenylmethoxycarbonyloxy)succinimide (1.40 g, 4.15 mmol) were added to a mixed solution of the intermediate (2-5) (2.00 g, 3.77 mmol) in tetrahydrofuran (19 ml)/water (19 mL), and the mixture was stirred for 1 hour. Saturated ammonium chloride aqueous solution (19 mL) and ethyl acetate (19 mL) were added to the reaction solution, and the organic layer was washed with saturated saline (5 mL), dried over sodium sulfate, and filtered. The filtrate was concentrated under reduced pressure, and the obtained residue was purified by silica gel column chromatography (eluent: hexane/ethyl acetate) to obtain 1.73 g of an example compound (1).

¹H-NMR (DMSOd₆, 400 MHz): δ = 11.58 (1H, s), 8.46 to 8.35 (1H, m), 7.88 (2H, d, J = 7.5 Hz), 7.70 (2H, d, 7.5Hz), 7.44 to 7.36 (2H, m), 7.35 to 7.25 (3H, m), 7.19 (1H, d, J = 8.3Hz), 6.85 to 6.67 (4H, m), 5.15 (2H, s), 4.92 (2H, s), 4.31 to 4.17 (3H, m), 3.90 to 3.81 (1H, m), 3.74 (3H, s), 3.71 (3H, s), 3.53 to 3.45 (1H, m), 3.45 to 3.38 (1H, M), 2.30 (3H, s), 2.24 (3H, s), 1.77 to 1.42 (4H, m), 1.30 to 1.21 (1H, m), 0.90 to 0.79 (1H, m)
ESI-MS(+) = 753.0, (-) = 751.1

### <Comparative compounds 1 and 2>

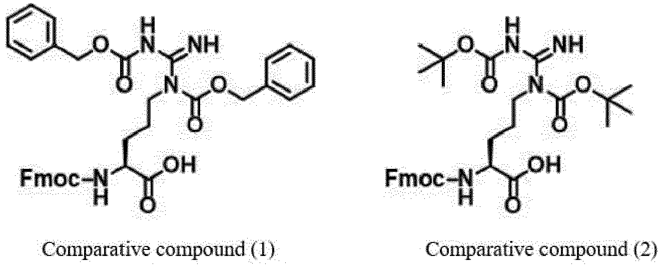

As Comparative Compounds 1 and 2, commercially available products manufactured by WATANABE CHEMICAL INDUSTRIES, LTD. were used.

### (Evaluation 1)

### <Deprotection rate>

With regard to the protected amino acid synthesized above, a deprotection ratio of a protected side chain site was determined as follows.

50 mg of the side chain-protected amino acid using the example compound 1 (protective group-forming reagent) or the side chain-protected amino acid using the comparative compounds 1 and 2 and Fmoc-Gly-OH (internal standard) in an equimolar amount of the side chain-protected amino acid were mixed, and then dichloromethane/trifluoroethanol/trifluoroacetic acid (100/10/1: vol ratio) was added thereto so that the substrate concentration was 0.025 M based on the side chain-protected amino acid, and the mixture was stirred at 30°C for 60 minutes.

5 µL of the reaction solution was dissolved in 400 µL of methanol (MeOH), and using Ultra Performance LC (ultra-performance liquid chromatography, manufactured by Waters Corporation, model number: ACQUITY), the deprotection ratio (%) was determined by quantifying the ratio of Fmoc-amino acid and Fmoc-Gly-OH produced by deprotecting the side chain-protected amino acid, and evaluated based on the following standard.

The columns and measurement conditions used for the ultra-performance liquid chromatography are shown below.
Column: manufactured by Waters Corporation, model number: BEH C18 1.7 µm, 2.1 mm × 30 mm
Flow rate: 0.5 mL/min
Solvent: solution A: 0.1% formic acid-water, solution B: 0.1% formic acid-acetonitrile
Gradient cycle: 0.00 min (solution A/solution B = 95/5), 2.00 min (solution A/solution B = 5/95), 3.00 min (solution A/solution B = 95/5)
Detection wavelength: 254 nm

### Regarding the evaluation of the deprotection rate, a case of "B" or higher was regarded as acceptable. The results are shown in Table 1.

It can be said that, as the deprotection ratio is higher, the deprotection rate is higher, which is suitable.

### -Evaluation standard-

"A": deprotection ratio was 90% or more.
"B": deprotection ratio was 50% or more and less than 90%.
"C": deprotection ratio was 10% or more and less than 50%.
"D": deprotection ratio was less than 10%.

**[Table 1]**

| | Compound | Deprotection rate |
|---|---|---|
| Example 1 | Example compound 1 | A |
| Comparative Example 1 | Comparative compound 1 | D |
| Comparative Example 2 | Comparative compound 2 | D |

From Table 1, it was found that the example compound 1 (protective group-forming reagent) used in Example 1 was excellent in deprotection rate compared to the comparative compounds of Comparative Examples 1 and 2. Therefore, it can be said that it is suitable for the synthesis of an acid-labile peptide. In addition, the example compound 1 was also excellent in sealing property.

### <Synthesis of protected peptide (5-residue peptide: Fmoc-MeNle-MeNle-Arg(X)-Cys(Mmt)-Gly-NH₂)>

Details of each abbreviation other than the above are shown below.
MeNle: N-methylnorleucine residue
Arg(X): arginine residue having the protective group according to the present invention
Cys(Mmt): Mmt-protected cysteine residue
Mmt: methoxytrityl group

### <Example 2>

### (Synthesis of Fmoc-Gly-NH-IndoTAG)

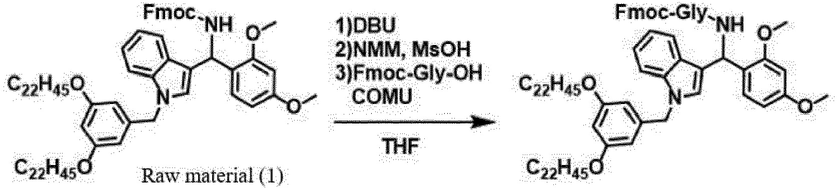

A raw material (1) (5.00 g, 4.02 mmol) synthesized according to Examples of WO2020/262259A was dissolved in tetrahydrofuran (40 mL), and diazabicycloundecene (DBU) (1.20 mL, 8.04 mmol) was added thereto and stirred. After the deprotection reaction was completed, N-methylmorpholine (0.906 mL, 8.24 mmol) and methanesulfonic acid (0.522 mL, 8.04 mmol) were added thereto, and Fmoc-Gly-OH (1.43 g, 4.82 mmol) and (1-cyano-2-ethoxy-2-oxoethylidenaminooxy)dimethylamino-morpholino-carbenium hexafluorophosphate (COMU) (2.07 g, 4.82 mmol) were added thereto and stirred. After the condensation reaction was completed, acetonitrile (400 mL) was added thereto and stirred, and then the precipitate was filtered, washed with acetonitrile, and dried under reduced pressure to obtain Fmoc-Gly-NH-IndoTAG (5.13 g, yield: 98.1%).

### (Synthesis of Fmoc-MeNle-MeNle-Arg(X)-Cys(Mmt)-Gly-NH-IndoTAG)

Using the Fmoc-Gly-NH-IndoTAG, a peptide sequence was extended by repeating the removal of the Fmoc group and the condensation reaction of the protected amino acid shown in Table 2. The X is the example compound 1.

**[Table 2]**

| Protected amino acid | Sequence | Yield [%] |
|---|---|---|
| Fmoc-Cys(Mmt)-OH | Fmoc-Cys(Mmt)-Gly - NH - IndoTag | 95.9 |
| Fmoc-Arg(X)-OH | Fmoc-Arg(X)-Cys(Mmt)-Gly - NH - IndoTag | 98.6 |
| Fmoc-MeNleu-OH | Fmoc-MeNle-Arg(X)-Cys(Mmt)-Gly-NH-IndoTag | 97.1 |
| Fmoc-MeNleu-OH | Fmoc-MeNle-MeNle-Arg(X)-Cys(Mmt)-Gly - NH - IndoTag | 98.3 |

### (Deprotection of peptide)

At room temperature, trifluoroacetic acid/hexafluoroisopropanol/dichloromethane (1/10/100: vol%, 2.0 mL), triisopropylsilane (10.0 molar equivalent), and 3,6-dioxa-1,8-octanedithiol (10.0 molar equivalent) were added to Fmoc-MeNle-MeNle-Arg(X)-Cys(Mmt)-Gly-NH-IndoTag (50.0 mg) and stirred at room temperature for 1 hour. The reaction solution was added dropwise to Tert-butylmethyl ether (20 mL) under ice-cooling to cause precipitation, and the supernatant removal and washing with Tert-butylmethyl ether were repeated to obtain Fmoc-MeNle-Arg(X)-Cys(Mmt)-Gly-NH-IndoTag (13.8 mg).
HPLC purity (220 nm): 84%
MS (ESI, m/Z): 810.4 (M + H)

According to the method for producing a peptide of the present disclosure, a peptide can be synthesized in a high yield. Particularly, since the protective group of the side chain can be deprotected even under weak acid conditions, an acid-labile peptide can be synthesized with high purity.

## Claims

1. A method for producing a peptide, comprising:
a peptide chain extending step of reacting a first amino acid or peptide in which a side chain is protected with a first protective group represented by Formula (1) with a second amino acid or peptide to obtain a third amino acid or peptide; and
a first deprotecting step of deprotecting the first protective group of the third amino acid or peptide,
wherein, in the first deprotecting step, the deprotection is performed using a deprotection solution having a trifluoroacetic acid content of 10% by mass or less, 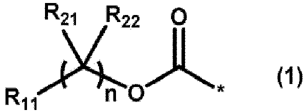
in the formula, R₁₁ is an aryl group having a substituent, R₂₁ and R₂₂ are each independently a hydrogen atom or a substituent, n is an integer of 1 to 6, and an asterisk represents a linking portion.

2. The method for producing a peptide according to claim 1,
wherein the trifluoroacetic acid content of the deprotection solution is 2% by mass or less.

3. The method for producing a peptide according to claim 1 or 2, further comprising:
a C-terminal protecting step of protecting a carboxy group or an amide group of an N-terminal protected amino acid or peptide with a C-terminal protective agent;
an N-terminal deprotecting step of deprotecting an N-terminal protective group of an N-terminal and C-terminal protected amino acid or peptide obtained in the C-terminal protecting step;
a peptide chain extending step of condensing an N-terminal of a C-terminal protected amino acid or peptide obtained in the N-terminal deprotecting step with an N-terminal protected amino acid or peptide; and
a C-terminal deprotecting step of deprotecting a C-terminal protective group,
wherein any of the C-terminal protected amino acid or peptide or the N-terminal protected amino acid or peptide is the first amino acid or peptide protected by the first protective group.

4. The method for producing a peptide according to any one of claims 1 to 3,
wherein the substituent of the aryl group in R₁₁ is an electron-donating group, and
a plurality of substituents may be the same or different from each other.

5. The method for producing a peptide according to claim 4,
wherein the electron-donating group is an amino group, an alkoxyalkyl group, an alkoxy group, or an alkyl group, which may further have a substituent.

6. The method for producing a peptide according to claim 4,
wherein the electron-donating group is an alkoxy group having 1 to 10 carbon atoms.

7. The method for producing a peptide according to any one of claims 1 to 6,
wherein the aryl group is a phenyl group or a naphthyl group.

8. The method for producing a peptide according to any one of claims 1 to 7,
wherein the first protective group is a protective group for a guanidino group of arginine.

9. A method for producing an amino acid or a peptide, comprising:
a protecting step of reacting a first amino acid or peptide with a protective group-forming reagent represented by Formula (2) to obtain a first protected amino acid or peptide in which a side chain is protected with a first protective group, 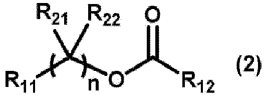 in the formula, R₁₁ is an aryl group having a substituent, R₂₁ and R₂₂ are each independently a hydrogen atom or a substituent, and n is an integer of 1 to 6.

10. Arginine or a compound containing an arginine residue, which has a protective group represented by Formula (1), 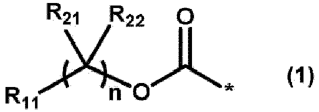 in the formula, R₁₁ is an aryl group having an electron-donating group, where a plurality of electron-donating groups may be the same or different from each other, R₂₁ and R₂₂ are each independently a hydrogen atom or a substituent, n is an integer of 1 to 6, and an asterisk represents a linking portion.

11. The arginine or the compound containing an arginine residue according to claim 10,
wherein the electron-donating group is an amino group, an alkoxyalkyl group, an alkoxy group, or an alkyl group, which may have a substituent.

12. The arginine or the compound containing an arginine residue according to claim 11,
wherein the electron-donating group is an alkoxy group having 1 to 10 carbon atoms.

13. The arginine or the compound containing an arginine residue according to any one of claims 10 to 12,
wherein the aryl group is a phenyl group or a naphthyl group.

14. The arginine or the compound containing an arginine residue according to any one of claims 10 to 13,
wherein the protective group is a protective group for a guanidino group of arginine.

15. The arginine or the compound containing an arginine residue according to any one of claims 10 to 14,
wherein the arginine or the compound further has a 9-fluorenylmethoxycarbonyl group.

16. A protective group-forming reagent represented by Formula (2), 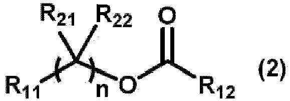 in the formula, R₁₁ is an aryl group which may have a substituent, R₁₂ is an aryl group which may have a substituent, a heteroaliphatic ring group, or a heteroaryl group, where the groups may be linked through an oxygen atom or a sulfur atom, R₂₁ and R₂₂ are each independently a hydrogen atom or a substituent, and n is an integer of 1 to 6.
